# EUROPEAN PATENT SPECIFICATION

(11) **EP 4 134 048 B1**
(45) Date of publication and mention of the grant of the patent: **08.05.2024**
(21) Application number: 21784875.3
(22) Date of filing: 30.03.2021
(51) Int. Cl.: A61F 2/95, A61F 2/24

(54) **LOADING TOOL AND SYSTEM FOR IMPLANT**
LADEWERKZEUG UND SYSTEM FÜR IMPLANTAT
OUTIL DE CHARGEMENT ET SYSTÈME POUR IMPLANT

(30) Priority: 07.04.2020 CN 202010266609
(43) Date of publication of application: 15.02.2023
(73) Proprietor: Shanghai Microport Cardioflow Medtech Co., Ltd., Shanghai 201203 (CN)
(72) Inventor: GU, Xiaojie, Shanghai 201203 (CN); SHI, Ruolin, Shanghai 201203 (CN); CHEN, Guoming, Shanghai 201203 (CN)
(74) Representative: Lavoix
(86) International application number: PCT/CN2021/084162
(87) International publication number: WO 2021/204028

(56) References cited:
- WO-A1-2019/179221
- CN-A- 106 361 467
- CN-A- 106 361 467
- CN-U- 209 661 884
- CN-U- 209 827 108
- CN-U- 212 415 989
- US-A1- 2015 112 431
- US-A1- 2019 053 900

## Description

### TECHNICAL FIELD

The present invention relates to the technical field of medical instruments and, in particular, to a loading tool and a loading system for an implant.

### BACKGROUND

Mitral regurgitation (MR) is a common disease or comorbidity in clinical practice. According to the latest epidemiological survey data from western developed countries such as the United States, among the elderly over 65 years old, mitral regurgitation is a kind of valvular diseases with the highest incidence. Presently, there is no authoritative epidemiological survey data in our country. However, there is no doubt about the huge number patients suffering from mitral regurgitation in our country. To date, surgical valve replacement or repair is the most preferred treatment for severe primary mitral regurgitation, including rheumatic and degenerative mitral regurgitation. However, for many elderly high-risk patients with multisystem disease, the surgical risk is high and the survival benefit is small. Data from Europe show that the surgical success rate of such patients is only 50%, and the surgical success rate of patients suffering from severe functional regurgitation is even lower to 16%. Minimally invasive interventional surgery, transcatheter mitral valve replacement (TMVR) can theoretically benefit high-risk patients who have lost the opportunity for surgery. Currently, there are more than 30 TMVR devices under development worldwide, of which more than ten species have entered the human trial stage, with more than 200 patients, gradually confirming its effect and feasibility, greatly advancing its progress. This method can greatly reduce the surgical injury to patients, and expand the scope of the population applicable to surgery.

Self-expanding mitral valve prosthesis is a common transcatheter mitral valve replacement device, and shape memory alloy material is usually used as the raw material of mitral valve prosthesis stent. Shape memory alloy materials, such as Nitinol, can achieve self-transformation between metallic martensite and austenite at specific temperatures. Taking advantage of this feature, the mitral valve prosthetic stent can be loaded into a smaller sheath at low temperature and transported to where the native mitral valve is in the heart; after release in vivo, the temperature of the mitral valve prosthetic stent gradually rises in the blood environment, which enable the stent to be self-expanding and be fixed in the working position to replace the native mitral valve.

Due to the large size of the native mitral valve annulus, the mitral valve prosthesis stent is required to have a large diameter. However, the structure of the ventricular native tissue is complex. In order to reduce or avoid an influence on the normal function of the ventricular native tissue, the ventricular section of the stent is generally short. This makes the mitral valve prosthesis stent to have a large size and a large diameter-to-axis ratio. Moreover, a large radial support is required, which makes it difficult to hold the mitral valve prosthetic stent and prone to serious distortion, deformation, unevenness, etc., resulting in damage to the stent, or even causing the stent to work abnormally. Therefore, a loading tool which is safe and efficient for compressing and holding mitral valve prosthesis stents is urgently needed.

CN106361467A or WO2019/179221A1 discloses a device that allows a compression in two steps with the help of one single work-piece.

### SUMMARY OF THE INVENTION

An object of the present invention is to provide a loading tool and a loading system for an implant, so as to solve the problem that the existing loading tools are difficult to apply to implants with large volume, implants with large diameter-to-axis ratio and implants with large radial support.

In order to solve the above technical problem, the present invention provides a loading tool for an implant, comprising a base, a first workpiece and a second workpiece;
wherein the base comprises a groove arranged along an axial direction of the base, the groove having an opening toward a first direction and configured for allowing an implant to be accommodated and placed therein;
the first workpiece comprising a first inner cavity extending through the first workpiece, the first inner cavity having a first end hole that has an inner diameter smaller than a maximum inner diameter of the groove; the first workpiece configured to detachably connect to the base, and the first end hole facing toward the first direction;
the second workpiece comprising a second inner cavity extending through the second workpiece, the second inner cavity having a second end hole that has an inner diameter smaller than the inner diameter of the first end hole; the second workpiece configured to detachably connect to the base or the first workpiece, and the second end hole facing toward the first direction;
wherein the groove, the first end hole and the second end hole are coaxially arranged in sequence in the first direction after being assembled and connected together.

Optionally, in the loading tool for an implant, the groove comprises an outer sidewall extending along the first direction.

Optionally, in the loading tool for an implant, the base comprises a sheath passageway extending through the base in the axial direction, the sheath passageway arranged internal to and coaxially with the outer sidewall.

Optionally, in the loading tool for an implant, the first inner cavity comprises a first neck section tapered from an end thereof for connecting with the base to the first end hole.

Optionally, in the loading tool for an implant, the second inner cavity comprises a second neck section tapered from an end thereof for connecting with the base or the first workpiece to the second end hole.

Optionally, in the loading tool for an implant, one of the first workpiece and the base comprises a first fastener, and the other one of the first workpiece and the base comprises a first receptacle matched with the first fastener, and wherein the first workpiece is connected to the base by a snap-in connection between the first fastener and the first receptacle.

Optionally, in the loading tool for an implant, the first workpiece comprises two or more first fasteners distributed in a circumferential direction thereof, each of the first fasteners extending to an end for connecting with the base; wherein the base comprises two or more first receptacles distributed in a circumferential direction of the base, each of the first receptacles extending through the base along the axial direction, so as to allow a corresponding one of the first fasteners to pass therethrough for snap-in connection; a circumferential width of the first receptacle is matched with the first fastener.

Optionally, in the loading tool for an implant, the first workpiece comprises two or more first guiding members distributed in the circumferential direction of the first workpiece, each of the first guiding members extending to an end for connecting with the base; the first receptacle further configured to allow the first guiding member to pass therethrough for snap-in connection; the circumferential width of the first receptacle matched with the first guiding member.

Optionally, in the loading tool for an implant, the first fastener protrudes from the base in a radial direction thereof, and the first receptacle is formed on the first workpiece and has an L-shape.

Optionally, in the loading tool for an implant, the second workpiece comprises a second fastener, and the base or the first workpiece comprises a second receptacle matched with the second fastener; or, the base or the first workpiece comprises a second fastener, and the second workpiece comprises a second receptacle matched with the second fastener; the second workpiece connected to the base or the first workpiece by a snap-in connection between the second fastener and the second receptacle.

Optionally, in the loading tool for an implant, the second workpiece comprises two or more second fasteners distributed in a circumferential direction of the second workpiece, each of the second fasteners extending to an end for connecting with the base; wherein the base comprises two or more second receptacles distributed in a circumferential direction of the base, each of the second receptacles extending through the base along the axial direction thereof, so as to allow a corresponding one of the second fasteners to pass therethrough for snap-in connection; a circumferential width of the second receptacle matched with the second fastener.

Optionally, in the loading tool for an implant, the second workpiece comprises two or more second guiding members distributed in the circumferential direction of the second workpiece, each of the second guiding members extending to an end for connecting with the base; the second receptacle further configured to allow the second guiding member to pass therethrough for snap-in connection; the circumferential width of the second receptacle matched with the second guiding member.

Optionally, in the loading tool for an implant, the second fastener protrudes from the first workpiece in a radial direction thereof, and the second receptacle is formed on the second workpiece and has an L-shape.

Optionally, in the loading tool for an implant, the first workpiece is connected with the base by threaded engagement; or, the second workpiece is connected with the base or the first workpiece by threaded engagement.

Optionally, the loading tool for an implant further comprises a diameter per-reducing workpiece that comprises a third inner cavity extending through the diameter per-reducing workpiece, the third inner cavity having a third end hole and a fourth end hole opposite to the third end hole, the third end hole having an inner diameter that is smaller than an inner diameter of the fourth end hole and not larger than the maximum inner diameter of the groove; the diameter per-reducing workpiece configured to pre-reduce a diameter of the implant; the third inner cavity comprising an inlet section, a buffer section and an outlet section, which are arranged in sequence from the fourth end hole to the third end hole; the buffer section having a drum shape.

Optionally, a ratio of the inner diameter of the third end hole to the inner diameter of the fourth end hole ranges from 0.4 to 0.6.

Optionally, in the loading tool for an implant, the base and/or the diameter per-reducing workpiece include(s) an anti-skid structure arranged along the circumferential direction of the base and/or the circumferential direction of the diameter per-reducing workpiece.

Optionally, in the loading tool for an implant, at least one of the base, the first workpiece, the second workpiece and the diameter per-reducing workpiece is transparent.

In order to solve the above technical problem, the present invention also provides a loading system for an implant, comprising:
a delivery device configured to deliver an implant to a target position; and
the above loading tool for an implant, configured to load the implant into the delivery device.

In conclusion, in the loading tool and loading system according to the present invention, the loading tool includes a base, a first workpiece and a second workpiece, and after the base, the first workpiece and the second workpiece are assembled and connected together, the groove of the base, the first end hole of the first workpiece and the second end hole of the second workpiece are coaxially arranged in sequence along the first direction, and the maximum inner diameter of the groove, the inner diameter of the first end hole and the inner diameter of the second end holes are successively reduced, so that the implant can be compressed step by step when passing through the first end hole and the second end hole in sequence. In addition, the end of the implant is allowed to be accommodated and placed in the groove, and the implant can be better fixed by the snap-in connection between the first workpiece and the base, and the stability of the implant during the compressing process is improved.

### BRIEF DESCRIPTION OF THE DRAWINGS

Those of ordinary skill in the art will appreciate that the accompanying drawings are provided for a better understanding of the present invention and do not constitute any limitation on the scope of the present invention.
Fig. 1 is a schematic diagram of a base according to Embodiment 1 of the present invention;
Fig. 2 is a schematic diagram of a first workpiece according to Embodiment 1 of the present invention;
Fig. 3 is a schematic diagram of a second workpiece according to Embodiment 1 of the present invention;
Fig. 4 is a schematic diagram of a diameter per-reducing workpiece according to Embodiment 1 of the present invention;
Fig. 5 is a schematic diagram showing an axial section of the diameter per-reducing workpiece according to Embodiment 1 of the present invention;
Figs. 6a-6d are schematic diagrams showing steps of using the loading tool according to Embodiment 1 of the present invention;
Fig. 7 is a schematic diagram of an anti-skid structure according to Embodiment 1 of the present invention;
Fig. 8 is a schematic diagram showing that the first workpiece is assembled with the base according to Embodiment 2 of the present invention;
Fig. 9 is a schematic diagram showing that the second workpiece is assembled with the first workpiece according to Embodiment 2 of the present invention;
Fig. 10 is a schematic diagram showing that the first workpiece is assembled with the base according to Embodiment 3 of the present invention;
Fig. 11 is a sectional view of the first workpiece and the base in Fig. 10; and
Fig. 12 is a schematic diagram showing an axial section of an ordinarily tapered diameter per-reducing workpiece according to Embodiment 1 of the present invention.

List of Reference Numerals:
100: base; 101: groove; 102: outer sidewall; 103: sheath passageway; 104: anti-skid structure; 105: base support; 106: first receptacle; 107: second receptacle; 108: first fastener;
200: first workpiece; 201: first end hole; 202: first neck section; 203: first fastener; 204: first guiding member; 205: the first receptacle; 206: second fastener;
300: second workpiece; 301: second end hole; 302: second neck section; 303: second fastener; 304: second guiding member; 305: second receptacle;
400: diameter per-reducing workpiece; 401: third end hole; 402: fourth end hole; 403: buffer section; 410: ordinarily tapered diameter per-reducing workpiece.

### DETAILED DESCRIPTION

To make the objectives, advantages and features of the present invention clearer, the present invention will be further described in detail below with reference to the drawings. It is noted that the figures are provided in a very simplified form not necessarily presented to scale, with their only intention to facilitate explaining the disclosed embodiments in a convenient and clear way. Furthermore, the structures shown in the drawings are often part of the actual structure. In particular, the figures generally give emphasis on different details and are accordingly drawn to different scales.

As used in this specification, the singular forms "a", "an", and "the" include plural referents, the term "or" is generally employed in its sense including "and/or", the term "proximal end" usually refers to the end close to the operator, the term "distal end" usually refers to the end close to the patient or the diseased site, the terms "one end" and "the other end" usually refer to the two opposite parts, and the terms "proximal end" and "distal end" usually refer to the two opposite parts, which not only include endpoints unless the content clearly indicates otherwise.

The core idea of the present invention is to provide a loading tool and a loading system for an implant, so as to solve the problem that the existing loading tools are difficult to apply to implants with large volume, implants with large diameter-to-axis ratio and implants with large radial support.

The following description is made with reference to the accompanying drawings.

### Embodiment 1

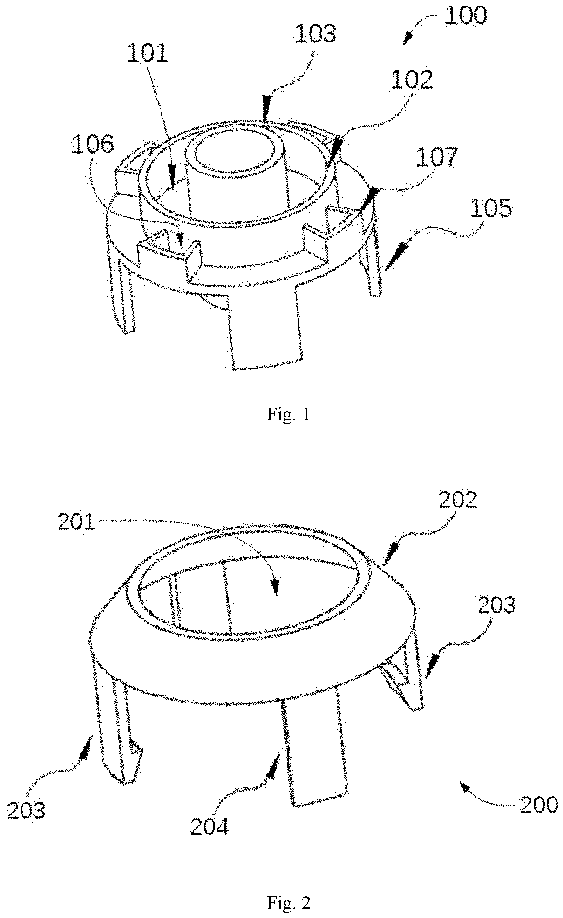

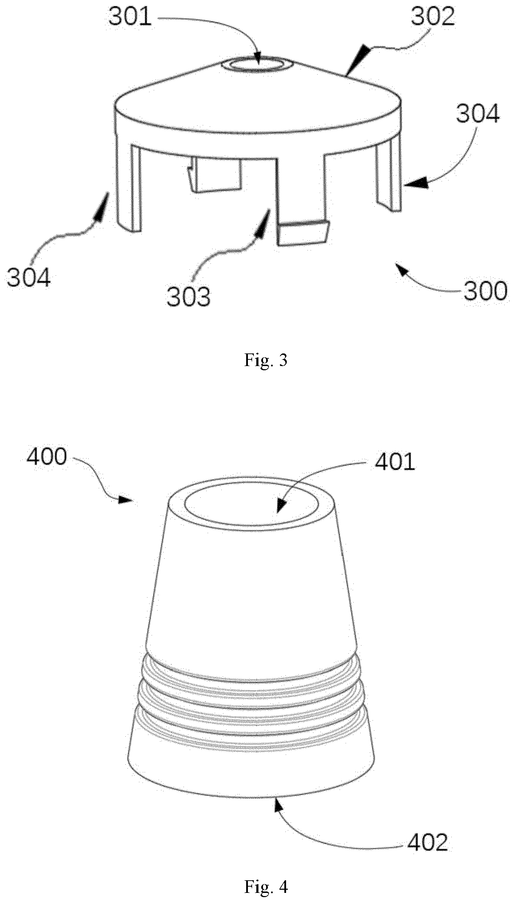

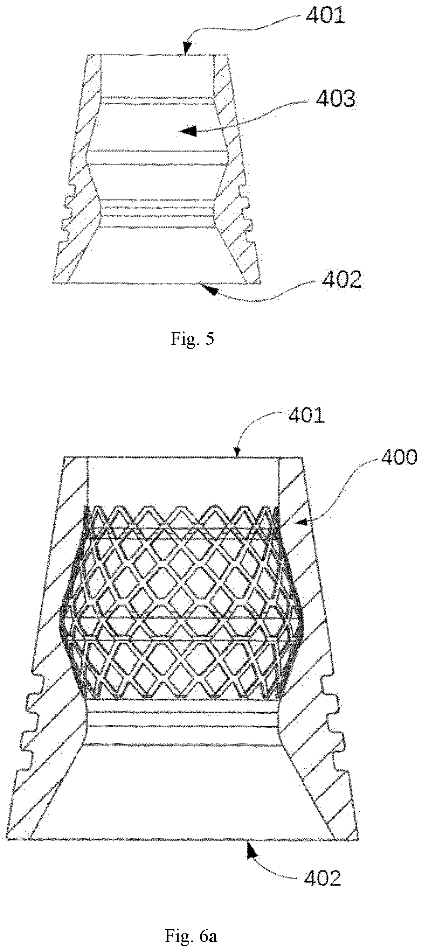

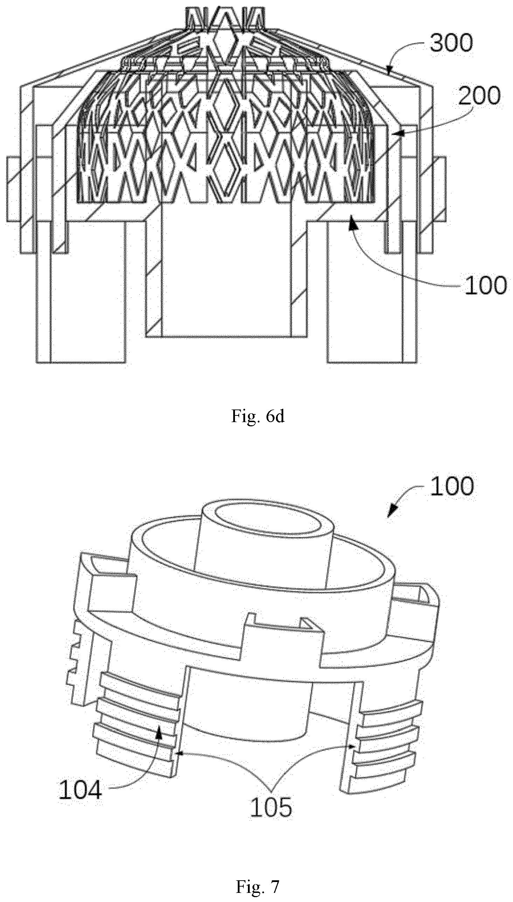

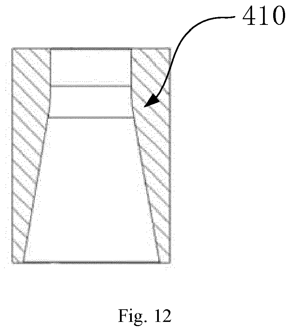

Please refer to Figs. 1-7 and 12, Fig. 1 is a schematic diagram of a base according to Embodiment 1 of the present invention; Fig. 2 is a schematic diagram of a first workpiece according to Embodiment 1 of the present invention; Fig. 3 is a schematic diagram of a second workpiece according to Embodiment 1 of the present invention; Fig. 4 is a schematic diagram of a diameter per-reducing workpiece according to Embodiment 1 of the present invention; Fig. 5 is a schematic diagram showing an axial section of the diameter per-reducing workpiece according to Embodiment 1 of the present invention; Figs. 6a-6d are schematic diagrams showing steps of using the loading tool according to Embodiment 1 of the present invention; Fig. 7 is a schematic diagram of an anti-skid structure according to Embodiment 1 of the present invention; and Fig. 12 is a schematic diagram showing an axial section of an ordinarily tapered diameter per-reducing workpiece according to Embodiment 1 of the present invention.

Embodiment 1 of the present invention provides a loading tool for an implant, which includes a base 100, a first workpiece 200 and a second workpiece 300. The base 100 includes a groove 101 extending along the axial direction of the base. The groove 101 has an opening toward a first direction and is configured for allowing an implant to be accommodated and placed therein. The first workpiece 200 includes a first inner cavity extending through the first workpiece. The first inner cavity has a first end hole 201 which has an inner diameter smaller than the maximum inner diameter of the groove 101. The first workpiece 200 is configured for detachably connecting with the base 100, and the first end hole 201 faces toward the first direction. The second workpiece 300 includes a second inner cavity extending through the second workpiece. The second cavity has a second end hole 301 which has an inner diameter smaller than the inner diameter of the first end hole 201. The second workpiece 300 is configured for detachably connecting with the base 100 or the first workpiece 200, and the second end hole 301 faces toward the first direction. After the base 100, the first workpiece 200 and the second workpiece 300 are assembled and connected together, the groove 101, the first end hole 201 and the second end hole 301 are coaxially arranged in sequence along the first direction. With this arrangement, the implant can be compressed step by step when passing through the first end hole 201 and the second end hole 301 in sequence. In addition, the groove 101 allows an end of the implant to be accommodated and placed therein, which improves the stability of the implant during the compressing process.

The following describes the loading tool according to the present invention by using an exemplary embodiment, and uses a mitral valve prosthesis as an example of the implant. Those skilled in the art may know that the implant is not limited to the mitral valve prosthesis/stent. Implants with a large volume, large diameter-to-axial ratio or large radial support, can all be compressed and held by the loading tool according to the present invention.

Please refer to Fig. 1 which is a schematic diagram of a base 100 according to Embodiment 1 of the present invention, the base 100 includes a groove 101 with an opening toward a first direction (i.e., an upward direction shown in Fig. 1). The groove 101 is substantially annular, and has a bottom surface facing toward the first direction. The bottom surface of the groove is configured to abut against the annular end surface of the mitral valve stent, so as to limit the displacement of the mitral valve stent along the axial direction of the base 100. Preferably, the groove 101 includes an outer sidewall 102 extending along the first direction. The arrangement of the outer sidewall 102 can effectively limit the radial degree of freedom of the mitral valve stent. In practice, when the mitral valve stent is accommodated and placed in the groove 101, it often abuts against the inner side of the outer sidewall 102. Further, the base 100 includes a sheath passageway 103 extending through the base in the axial direction thereof. The sheath passageway 103 is arranged internal to the outer sidewall 102 and coaxially with the outer sidewall 102. The sheath passageway 103 may be a tube defining an inner space allowing the sheath in the delivery device to pass through, thereby facilitating the loading of the mitral valve stent into the sheath. The spaced area between the sheath passageway 103 and the outer sidewall 102 forms the opening of the groove 101, which can be used for the placement of the mitral valve stent.

Please refer to Fig. 7, optionally, the base 100 further includes an anti-skid structure 104 which may be provided on the base 100 along the circumference thereof to increase the anti-skid performance and facilitate the grasp of the operator. The anti-skid structure 104 can be, for example, three raised anti-skid edges, or a frosted surface. In the examples shown in Figs. 1 and 7, the base 100 includes a plurality of circumferentially distributed base supports 105 extending along the axial direction. The plurality of base supports 105 are preferably distributed centrosymmetrically or axisymmetrically, so as for balancing forces. The axial length of the base supports 105 is about 1/4~2/5 of the total axial length of the base 100. As shown in Fig. 7, the anti-skid structure 104 can be provided on the base supports 105. The plurality of base supports 105 which are arranged separately are mainly configured to provide space for the snap-in connection between the base 100 and the first workpiece 200 or the second workpiece 300, which will be described in the following parts in relation to the snap-in connection. Of course, the arrangement of the plurality of base supports 105 is merely an example, and the base 100 is not limited to being provided with the plurality of base supports 105. For example, the ends of the base supports 105 away from the first direction may also be a circumferentially closed cylinder having a plurality of hollow cavities which may provide operating space for the snap-in connection. It is not limited to this in the embodiment.

Please refer to Fig. 2 which is a schematic diagram of a first workpiece 200 according to Embodiment 1 of the present invention, the first workpiece 200 includes a first inner cavity extending through the first workpiece 200 in an axial direction thereof. The first inner cavity has a first end hole 201. Since the groove 101 of the base 100 is annular and has a certain width, the maximum inner diameter of the groove 101 can be considered as the inner diameter of the outer sidewall 102. The inner diameter of the first end hole 201 is smaller than the maximum inner diameter of the groove 101, so that the mitral valve stent can be compressed for the first time when passing through the first end hole 201. Preferably, the first inner cavity includes a first neck section 202 which is tapered from an end for connecting with the base 100 (hereinafter, referred to as the first expanded end for ease of description) to the first end hole 201. That is, the first neck section 202 is gradually constricted in the direction in which the mitral valve stent is allowed to pass through. In this configuration, when the mitral valve stent passes through the first inner cavity of the first workpiece 200, the mitral valve stent will be gradually compressed, and then the mitral valve stent will go out of the first end hole 201 to complete the first compression. Further, the inner diameter of the first expanded end is not less than the maximum inner diameter of the groove 101, so that the mitral valve stent can be accommodated in the first inner cavity. Preferably, the inner diameter of the first expanded end is same as the outer diameter of the outer sidewall 102 of the groove 101, which allows the base 100 to be nested in the first workpiece 200, thereby achieving a reduced size. Preferably, the ratio of the inner diameter of the first end hole 201 to the inner diameter of the first expanded end ranges from 0.85 to 0.95. After the first workpiece 200 and the base 100 are assembled and connected, the first neck section 202 can secure the mitral valve stent on the first workpiece 200 and the base 100, and the inclined surface of the first neck section 202 provides the mitral valve stent with a push force away from the first direction, thereby facilitating the secure of the mitral valve stent, and resulting in the ventricular end of the mitral valve stent being compressed for the first time.

Please refer to Fig. 3 which is a schematic diagram of a second workpiece 300 according to Embodiment 1 of the present invention, the second workpiece 300 includes a second inner cavity extending through the second workpiece 300 in the axial direction thereof. The second inner cavity has a second end hole 301 which has an inner diameter smaller than the inner diameter of the first end hole 201, so that the mitral valve stent can be compressed for the second time when passing through the second end hole 301. Preferably, the second inner cavity includes a second neck section 302 which is tapered from an end for connecting with the base 100 or the first workpiece 200 (hereinafter, referred to as the second expanded end for ease of description) to the second end hole 301. That is, the second neck section 302 is gradually constricted along the direction in which the mitral valve stent is allowed to pass through. In this configuration, when the mitral valve stent having been compressed for the first time passes through the second inner cavity of the second workpiece 300, it will pass through the second end hole 301 and be gradually compressed, during which the mitral valve stent can be sized to be suitable for the snap-in connection with a delivery device configured to deliver the mitral valve stent to a target position; and when the second workpiece 300 is completely fixed with the first workpiece 200 or the base 100, the second compression is completed. Further, the inner diameter of the second end hole 301 is sized to be able to cooperate with the end of the sheath of the delivery device, so as to ensure that the sheath can pass through the second end hole 301. The inner diameter of the second expanded end is larger than the outer diameter of the first workpiece 200 so that the second workpiece 300 can be assembled with the first workpiece 200 or the base 100.

The above configuration allows, in actual use, the mitral valve stent to be secured by the first workpiece 200 and the base 100, and pass through the first end hole 201 of the first workpiece 200 and the second end hole 301 of the second workpiece 300 in sequence along the first direction, so as to complete the two compressions.

In order to facilitate the detachable assembly and connection between the base 100, the first workpiece 200 and the second workpiece 300, the first embodiment mainly employs the snap-in connection. The snap-in connection is good for maintaining the coaxality and reducing damage to the mitral valve stent, and also allows a simple operation. It should be understood that, after the base 100, the first workpiece 200 and the second workpiece 300 are assembled, it is merely necessary to ensure that the groove 101, the first end hole 201 and the second end hole 301 are coaxially arranged in sequence along the first direction. In some embodiments, the first workpiece 200 may be provided with some through holes, and a part of the second workpiece 300 (such as a fastener) may pass through the through holes of the first workpiece 200 so as to be connected with the base 100. Therefore, the arrangements of the base 100, the first workpiece 200 and the second workpiece 300 is not limited here. Those skilled in the art are able to create various kinds of connections between the base 100, the first workpiece 200 and the second workpiece 300 according to actual situations.

Optionally, one of the first workpiece 200 and the base 100 includes a first fastener, the other includes a first receptacle matched with the first fastener. The first workpiece 200 can be connected to the base 100 by a snap-in connection between the first fastener and the first receptacle. Preferably, in some embodiments, the first workpiece 200 includes two or more first fasteners 203 distributed in the circumferential direction, and each of the first fasteners 203 extends toward an end for connecting with the base 100. The base 100 includes two or more first receptacles 106 distributed in the circumferential direction, and the first receptacles 106 extend through the base 100 in the axial direction thereof to allow the first fastener 203 to pass through for the snap-in connection. The circumferential width of the first receptacles 106 is suitable for the first fastener 203. In the example shown in Figs. 1 and 2, the base 100 includes four first receptacles 106 evenly distributed in the circumferential direction, so as to balance the force and allow a more stable connection between the first workpiece 200 to the base 100. The first workpiece 200 includes two first fasteners 203 symmetrically distributed in the circumferential direction, and each of the first fasteners 203 has a triangular engaging tooth that inwardly protrudes so as to be engaged with the corresponding first receptacle 106. It can be understood that the first fastener 203 has a certain elasticity so as to be easily engaged and disengaged. Further, the circumferential width of the first receptacle 106 is suitable for the first fastener 203, which allows the first receptacle 106 to limit the circumferential displacement of the first fastener 203, and can prevent form creating circumferential rotations after the first workpiece 200 is assembled with the first workpiece 200. Of course, in some other embodiments, it is also possible to provide the first receptacles on the first workpiece 200, and provide the first fastener on the base 100, and those skilled in the art can perform different configurations.

Further, the first workpiece 200 includes two or more first guiding members 204 distributed in the circumferential direction, and each of the first guiding members 204 extends toward an end for connecting with the base. The first receptacles 106 are also configured for allowing the first guiding members 204 to pass therethrough for locating the same. The circumferential width of the first receptacle 106 is suitable for the first guiding member 204. In the example shown in Figs. 1 and 2, the first workpiece 200 includes two first guiding members 204 distributed symmetrically. Preferably, the two first guiding members 204 and the two first fasteners 203 are distributed at an angle of 90°. That is, in the circumferential direction, the first guiding members 204 and the first fasteners 203 are sequentially arranged at intervals. Both the first guiding members 204 and the first fasteners 203 can be inserted into the first receptacles 106, The first guiding members 204 are merely configured to locate the first workpiece to circumferentially limit the same, and the first fasteners 203 are configured not only to circumferentially limit the first workpiece but also to limit the axial displacement of the first workpiece 200 relative to the base 100. Since the diameter of the mitral valve stent is large, the diameter of the loading tool for the mitral valve stent is also large. The arrangement of the first guiding member 204 is more conducive to enhancing the coaxiality between the first workpiece 200 and the base 100, thereby ensuring the coaxiality thereof with the mitral valve stent. This reduces the possibility of damage to the mitral valve stent. Optionally, the number of the first receptacles 106 is not less than the sum of the numbers of the first fasteners 203 and the first guiding members 204. It can be understood that the numbers of the first fasteners 203, the first guiding members 204 and the first receptacles 106 are not limited to the numbers mentioned in the above example, and the distribution angle of the first guiding members 204 and the first fasteners 203 is not limited to 90°. For example, each of the first guiding members 204 and the corresponding one of the first fasteners 203 can be arranged in parallel at the same circumferential position on the first workpiece 200. In this configuration, the first guiding member 204 and the first fastener 203 can pass through a same first receptacle 106, thereby further saving the space.

Optionally, the second workpiece 300 includes second fasteners, and the base 100 or the first workpiece 200 includes second receptacles suitable for the second fasteners. Alternatively, the base 100 or the first workpiece 200 includes second fasteners, and the second workpiece 300 includes second receptacles suitable for the second fasteners. The second workpiece 300 can be connected to the base 100 or the first workpiece 200 by the snap-in connection between the second fasteners and the second receptacles. In practice, the second workpiece 300 can be directly connected to the base 100 by a snap-in connection, or can be indirectly connected to the base 100 through being connected to the first workpiece 200 by a snap-in connection. Preferably, in some embodiments, the second workpiece 300 includes two or more second fasteners 303 distributed in the circumferential direction, and each of the second fasteners 303 extends toward an end for connecting with the base. The base 100 includes two or more second receptacles 107 distributed along the circumferential direction. The second receptacles 107 pass through the base 100 along the axial direction thereof and are configured for allowing the second fasteners 303 to pass therethrough for creating a snap-in connection. The circumferential width of the second receptacles 107 is suitable for the second fasteners 303. In the example shown in Figs. 1 and 3, the base 100 includes four second receptacles 107 evenly distributed in the circumferential direction, and the circumferential positions of the second receptacles 107 are the same as the circumferential positions of the first receptacles 106. In practice, a same through hole can serve as both the first receptacle 106 and the second receptacle 107, and the inner sidewall of the through hole serves as the first receptacle 106 to be connected with the first fastener 203 by snap-in connection while the outer sidewall of the through hole serves as the second receptacle 107 to be connected with the second fastener 303 by snap-in connection, thereby not only saving the space, but also facilitating the identification and manipulation by the operator. The second workpiece 300 includes two second fasteners 303 distributed symmetrically, and each of the second fasteners 303 has a triangular-shaped engaging tooth protruding outward, so as to be connected with the second receptacle 107 by snap-in connection. The circumferential width of the second receptacle 107 is suitable for the second fastener 303, which allows limiting the circumferential rotation of the second workpiece 300. Preferably, the through holes forming the first receptacles 106 and the second receptacles 107 are closely attached to the outside of the outer sidewall 102 of the groove 101 to enhance the overall connection strength and reduce the size of the base 100. More preferably, the inner side surface of the second receptacle 107 has a certain slope relative to the axis of the base 100, and the cross section of the second receptacle 107 is triangular or trapezoidal, so as to be easily connected with the second fastener 303 by snap-in connection. Of course, in some other embodiments, the first receptacle 106 and the second receptacle 107 are not limited to be formed in a same through hole, and they can also be provided independently. In addition, it is also possible to form the second receptacles on the second workpiece 300, and to form the second fasteners on the base 100, and those skilled in the art can perform different configurations.

Preferably, the second workpiece 300 includes two or more second guiding members 304 distributed in the circumferential direction, and each of the second guiding members 304 extends toward an end for connecting with the base 100. The second receptacles 107 are also configured for allowing the second guiding members 304 to pass therethrough for locating the same. The circumferential width of the second receptacle 107 is suitable for the second guiding member 304. In the example shown in Figs. 1 and 3, the second workpiece 300 includes two second guiding members 304 distributed symmetrically. Preferably, the two second guiding members 304 and the two second fasteners 303 are distributed at an angle of 90°. That is, in the circumferential direction, the second guiding members 304 and the second fasteners 303 are arranged in sequence at intervals. Optionally, the number of the second receptacles 107 is not less than the sum of the numbers of the second fasteners 303 and the second guiding members 304. For the principle of arrangement of the second guiding members 304, reference may be made to the first guiding members 204, which will not be repeated here.

Preferably, the loading tool for an implant further includes a diameter per-reducing workpiece 400 including a third inner cavity extending through the diameter per-reducing workpiece. The third inner cavity has a third end hole 401 and a fourth end hole 402 opposite to the third end hole 401. The inner diameter of the third end hole 401 is smaller than the inner diameter of the fourth end hole 402, and the inner diameter of the third end hole 401 is not greater than the maximum inner diameter of the groove 101. The diameter pre-reducing workpiece 400 is configured to pre-reduce the diameter of the implant. Since the mitral valve stent has a large diameter, large volume and large diameter-to-axis ratio, it is preferable to use the pre-diameter workpiece 400 to pre-reduce the diameter of the mitral valve stent to 40%~60% of the original radial size before placing the mitral valve stent into the groove 101 of the base 100. It can be understood that the inner diameter of the fourth end hole 402 is close to the original radial size of the mitral valve stent, and the inner diameter of the fourth end hole 402 is preferably 0.9-1.1 times the diameter of the ventricular end of the mitral valve stent. The mitral valve stent passes through the third inner cavity through the fourth end hole 402, and passes through the third end hole 401, so as to achieve pre-reduction in diameter. Optionally, the ratio of the inner diameter of the third end hole 401 to the inner diameter of the fourth end hole 402 ranges from 0.4 to 0.6. The inner diameter of the third inner cavity generally decreases in the direction from the fourth end hole 402 to the third end hole 401, so as to realize the pre-reduced diameter of the mitral valve stent.

Referring to Figs. 4 and 5, in an exemplary embodiment, the third inner cavity includes an inlet section, a buffer section 403 and an outlet section arranged in sequence from the fourth end hole 402 to the third end hole 401. The buffer section 403 has a drum shape. Optionally, the inlet section has a tapered shape that gradually decreases from the fourth end hole 402 to the third end hole 401, and the outlet section has a cylindrical shape or a tapered shape that gradually decreases from the fourth end hole 402 to the third end hole 401. Preferably, the outlet section has a cylindrical shape, so that the mitral valve stent with a per-reduced diameter can be smoothly pulled out of the outlet section. Generally, when the tapered diameter per-reducing workpiece is pre-reducing the diameter of the mitral valve stent, it is likely that the mitral valve stent may pop out under extrusion pressure from the sidewall of the diameter per-reducing workpiece because the mitral valve stent is only subject to a pulling force directing the smaller end of the tapered shape. This may lead to a failure in pre-reducing the diameter of the mitral valve stent, or even a damage to the mitral valve stent. For this reason, the diameter per-reducing workpiece 400 provided in this embodiment is defined with the buffer section 403. In an aspect, after the mitral valve stent gradually enters the buffer section 403, the pressures from the inner walls of the buffer section 403 to the mitral valve stent are offset during the process of the mitral valve stent entering the outlet section, which allows a reduced pulling force required for traction, thereby reducing the damage to the mitral valve stent. In a further aspect, after the mitral valve stent enters the buffer section 403, the mitral valve stent can be temporarily fixed therein, so as to facilitate the adjustment of the shape of the mitral valve stent. Optionally, the inlet section, the buffer section 403 and the outlet section may be connected by a smooth transition, or may be directly connected at the corners. Preferably, the inner diameters of both ends of the buffer section 403 may be the same, or the inner diameter of the end connected to the inlet section may be larger than the inner diameter of the end connected to the outlet section. More preferably, the axial length of the buffer section 403 ranges from 0.8 to 1.2 times the axial length of the corresponding mitral valve stent to be reduced in diameter.

It should be noted that it is also possible to use an ordinarily tapered diameter per-reducing workpiece without the buffer section 403, as shown in Fig. 12, to pre-reduce the diameter of the mitral valve stent if the ordinarily tapered diameter per-reducing workpiece can meet the requirements for the diameter pre-reducing process.

Preferably, the diameter per-reducing workpiece 400 includes an anti-skid structure arranged along the circumferential direction of the diameter per-reducing workpiece 400. The anti-skid structure may be similar to the anti-skid structure on the base 100. For example, the anti-skid structure may be implanted as three raised anti-skid edges or a frosted surface to increase the anti-skid performance and facilitate the grasp of the operator.

In practice, the base 100, the first workpiece 200, the second workpiece 300 and the diameter pre-reducing workpiece 400 can be made in a variety of ways, including but not limited to the following cases where the base 100, the first workpiece 200, the second workpiece 300 or the diameter per-reducing workpiece 400 are respectively formed integrally by cutting technology, such as formed by cutting a polycarbonate (PC) bar using a lathe; or, the base 100, the first workpiece 200, the second workpiece 300 and the diameter per-reducing workpiece 400 are respectively formed integrally by 3D printing technology. Of course, one or more of the base 100, the first workpiece 200, the second workpiece 300 or the diameter pre-reduced workpiece 400 may be formed by cutting technology, and the other may be formed by 3D printing technology. For example, the material for the 3D printing technology can be at least one of transparent PC material, PMMA, PP, PE, MBS and PS material. Optionally, at least one of the base 100, the first workpiece 200, the second workpiece 300 and the diameter per-reducing workpiece 400 is transparent, for example, is made of a transparent PC material by cutting or 3D printing technology, so that the shape of the mitral valve stent can be observed in real time during the diameter pre-reducing process.

The steps for using the loading tool provided in this embodiment will be described below with reference to Figs. 6a to 6d.

Please refer to Fig. 6a, which is a schematic diagram showing that the diameter pre-reducing workpiece 400 is used to pre-reduce the diameter of the mitral valve stent. In practice, the fixing member (e.g., the lug) of the mitral valve stent is fixed by a traction means (e.g., a suture) in a low temperature environment (e.g., an ice-water bath), and the mitral valve stent is pulled to remove through the third inner cavity of the diameter per-reducing workpiece 400 in a direction from the fourth end hole 402 to the third end hole 401 of the diameter per-reducing workpiece 400. As a result, the diameter pre-reducing process is complete. When the mitral valve stent is in the position as shown in Fig. 6a, the mitral valve stent is pressed at the upper and lower parts thereof by the buffer section 403 and thus fixed in the buffer section 403. At this time, the circumferential uniformity of the mitral valve stent can be adjusted by using the traction means. In addition, the fixed configuration is also beneficial to the shape stability of the metal of the mitral valve stent. After the pre-compressing process, the mitral valve stent can be compressed to 40%-60% of the original diameter.

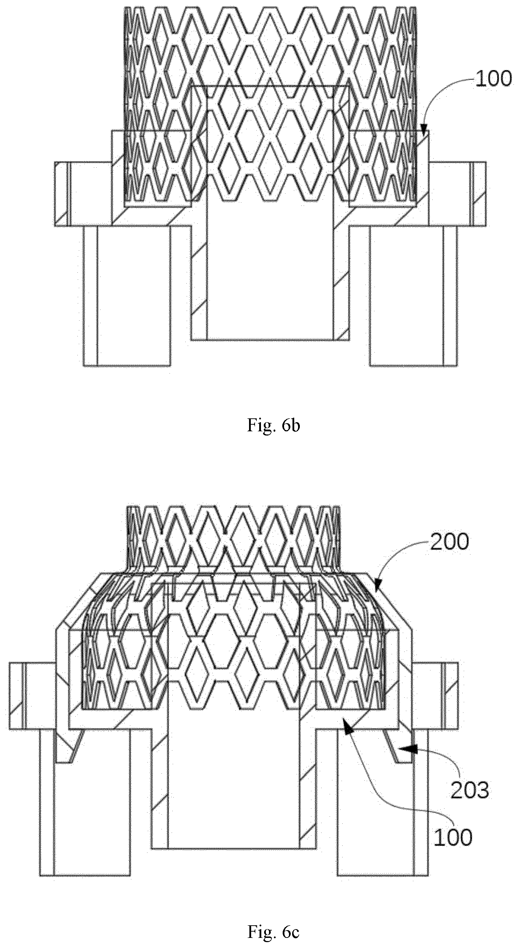

Please refer to Fig. 6b, which is a schematic diagram showing that the mitral valve stent is placed into the base 100. The mitral valve stent with a pre-reduced diameter is placed in the groove 101.

Please refer to Fig. 6c, which is a schematic diagram showing that the first workpiece 200 and the base 100 are assembled and connected with each other. After the mitral valve stent is placed in the groove 101, the first fastener 203 of the first workpiece 200 is inserted into the first receptacle 106 of the base 100 so that they are fixed together by snap-in connection. At this time, the mitral valve stent has been compressed for the first time at the ventricular end (i.e., the upper end shown in Fig. 6c) thereof by the first workpiece 200, and the ventricular end of the mitral valve stent protrudes out from the first end hole 201. The mitral valve stent is also fixed by the first neck section 202 of the first workpiece 200. During this process, the shape of the mitral valve stent or the membrane thereon can be adjusted.

Please refer to Fig. 6d, which is a schematic diagram showing that the second workpiece 300 and the base 100 are assembled and connected with each other. After the first work piece 200 is assembled and connected to the base 100, the second fastener 303 of the second work piece 300 is inserted into the second receptacle 107 of the base 100 so that they are fixed together by snap-in connection. At this time, the mitral valve stent has been compressed for the second time at the ventricular end (i.e., the upper end shown in Fig. 6d) thereof by the second workpiece 300, and the ventricular end of the mitral valve stent protrudes out from the second end hole 301. Further, the fixing member (e.g., the lug) of the mitral valve stent is fixed to the fixing component (e.g., the fixing head) of the delivery device, and then the entire loading tool is pulled in the opposite direction (as shown in the lower part shown in Fig. 6d), so that the sheath of the delivery device is moved until the mitral valve stent is completely covered by the sheath. As a result, the mitral valve stent is finally compressed and loaded.

The present embodiment also provides a loading system for an implant based on the above loading tool for an implant. The loading system includes a delivery device for delivering an implant to a target position and the above-mentioned loading tool configured to load the implant into the delivery device.

### Embodiment 2

The loading tool and loading system for an implant according to Embodiment 2 are basically the same as the loading tool and loading system for an implant according to Embodiment 1 of the present invention, and only the differences will be described below.

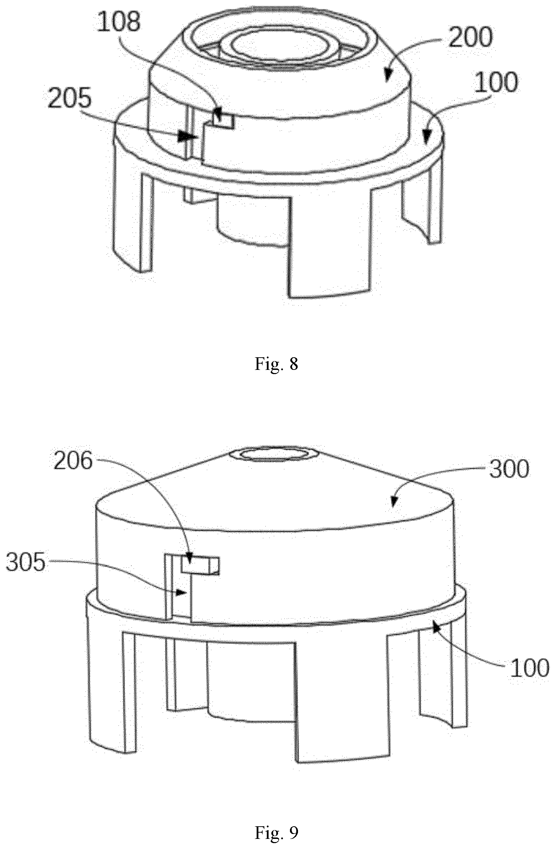

Reference is now made to Figs. 8 and 9. Fig. 8 is a schematic diagram showing that the first workpiece is assembled with the base according to Embodiment 2 of the present invention. Fig. 9 is a schematic diagram showing that the second workpiece is assembled with the first workpiece according to Embodiment 2 of the present invention.

In the loading tool for an implant according to Embodiment 2 of the present invention, the first workpiece 200, the second workpiece 300 and the base 100 are assembled in a way different from that in Embodiment 1. Specifically, in Embodiment 2, the first workpiece 200 is connected with the base 100 by rotational snap-in connection, and the second workpiece 300 is connected with the first workpiece 200 by rotational snap-in connection.

In an exemplary embodiment, the base 100 is provided with a first fastener 108, the first workpiece 200 is defined with a first receptacle 205, and the first fastener 108 protrudes from the base 100 in the radial direction thereof. The first receptacle 205 is formed on the first workpiece 200 and has an L-shape. Optionally, the first fastener 108 is preferably fixedly arranged on the outer side of the outer sidewall 102 of the groove 101; an end of the first workpiece 200 away from the first end hole 201 has a cylindrical extension, and the extension has an inner diameter preferably matched with the outer diameter of the outer sidewall 102 of the groove 101 so as to be sleeved over the outer sidewall 102; the L-shaped first receptacle 205 includes an axial section and a circumferential section; the axial section extends from the opening end of the extension towards the first end hole 201, and the circumferential section is connected to an end of the axial section close to the first end hole 201 and extends along the circumference of the first workpiece 200. The size of the first receptacle 205 is matched with the size of the first fastener 108. In actual use, the first workpiece 200 is coaxially aligned with the base 100, the first fastener 108 is aligned with the axial section of the first receptacle 205, then the first workpiece 200 is pushed toward the base 100 until the first fastener 108 is locked into the end of the axial section close to the first end hole 201, the first workpiece 200 is then rotated along the extending direction of the circumferential section, so that the snap-in connection between the first workpiece 200 and the base 100 is complete.

Further, the protruding first fastener 108 has a thickness matched with the wall thickness of the extension of the first workpiece 200. After the first workpiece 200 is assembled with the base 100, the first fastener 108 will not protrude out of the extension of the first workpiece 200. The first workpiece 200 is provided with a second fastener 206, and the second workpiece 300 is defined with a second receptacle 305. The second fastener 206 protrudes radially from the first workpiece 200. The second receptacle 305 is formed on the second workpiece 300 and has an L-shape. Optionally, the second fastener 206 is arranged on the outer side of the extension of the first workpiece 200. The structure of the second receptacle 305 is similar to that of the first receptacle 205, and reference may be made to the first receptacle 205. The description about the second receptacle 305 will not be repeated here. Preferably, the base 100 includes two or more first fasteners 108, and the first workpiece 200 includes two or more second fasteners 206. All the first fasteners 108 are evenly distributed around the circumference of the base 100, and all the second fasteners 206 are evenly distributed around the circumference of the first workpiece 200. Correspondingly, the numbers of the first receptacles 205 and the second receptacles 305 are not less than the numbers of the first fasteners 108 and the second fasteners 206, so as for snap-in connection.

The base 100, the first workpiece 200 and the second workpiece 300 can be easily connected or detached by a rotational snap-fit connection, and the connection ensures high coaxiality and high reliability. For other processes of using the loading tool to compress the implant step by step, reference may be made to Embodiment 1, which will not be repeated here.

### Embodiment 3

The loading tool and loading system for an implant according to Embodiment 3 are basically the same as the loading tool and loading system for an implant according to Embodiment 1 of the present invention, and only the differences will be described below.

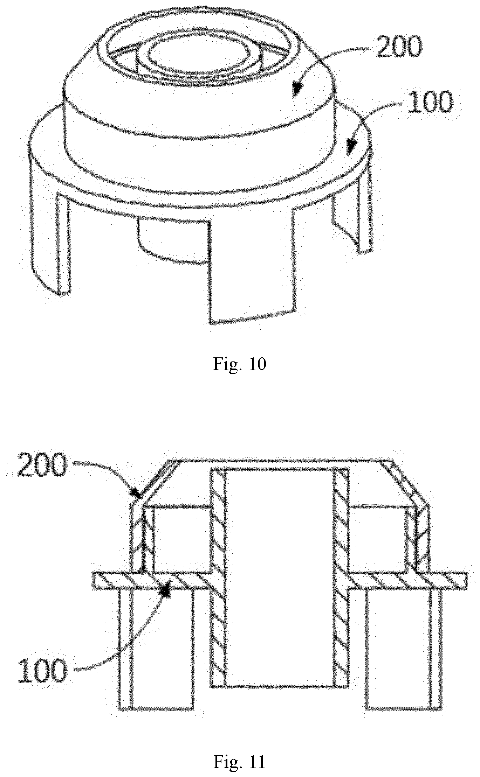

Please refer to Figs. 10 and 11. Fig. 10 is a schematic diagram showing that the first workpiece is assembled with the base according to Embodiment 3 of the present invention. Fig. 11 is a sectional view of the first workpiece and the base in Fig. 10.

In the loading tool for an implant according to Embodiment 3 of the present invention, the first workpiece 200, the second workpiece 300 and the base 100 are assembled in a way different from that in Embodiment 1. Specifically, in Embodiment 3, the first workpiece 200 and the base 100 are connected by threaded engagement, and the second workpiece 300 and the first workpiece 200 are connected by threaded engagement. Of course, in some other embodiments, it is also possible that either the connection between the first workpiece 200 and the base 100 or the connection between the second workpiece 300 and the first workpiece 200 is realized by threaded engagement.

In an exemplary embodiment, an end of the first workpiece 200 away from the first end hole 201 has a cylindrical extension, and the extension has an inner diameter preferably matched with the outer diameter of the outer sidewall 102 of the groove 101 so as to be sleeved over the outer sidewall 102; the inside of the extension and the outside of the outer sidewall 102 are respectively threaded so that they are matched with each other, and the first workpiece 200 can be connected to the base 100 by rotation. Further, the inside of the second workpiece 300 and the outside of the extension of the first workpiece 200 are respectively threaded so that they are matched with each other. As a result, the second workpiece 300 can be connected with the first workpiece 200 by thread engagement.

The base 100, the first workpiece 200 and the second workpiece 300 can be easily connected or detached by means of threaded engagement, and the connection ensures high coaxiality and high reliability. For other processes of using the loading tool to compress the implant step by step, reference may be made to Embodiment 1, which will not be repeated here.

It should be noted that the implementations of the above-mentioned several embodiments can be used in combination, for example, the first workpiece 200 and the base 100 can be connected by the rotational snap-in connection according to Embodiment 2, and the second workpiece 300 and the first workpiece 200 can be connected by threaded engagement according to Embodiment 3, and those skilled in the art can choose and use them in combination according to the above description.

In conclusion, in the loading tool and loading system according to the present invention, the loading tool includes a base, a first workpiece and a second workpiece, and after the base, the first workpiece and the second workpiece are assembled and connected together, the groove of the base, the first end hole of the first workpiece and the second end hole of the second workpiece are coaxially arranged in sequence along the first direction, and the maximum inner diameter of the groove, the inner diameter of the first end hole and the inner diameter of the second end holes are successively reduced, so that the implant can be compressed step by step when passing through the first end hole and the second end hole in sequence. In addition, the end of the implant is allowed to be accommodated and placed in the groove, and the implant can be better fixed by the snap-in connection between the first workpiece and the base, and the stability of the implant during the compressing process is improved.

The above description is only for the description of preferred embodiments of the present invention, and is not intended to limit the scope of the present invention. Any changes and modifications made by those skilled in the art according to the above disclosure are all within the protection scope of the appended claims.

## Claims

1. A loading tool for an implant, comprising a base (100), a first workpiece (200) and a second workpiece (300);
wherein the base (100) comprises a groove (101) arranged along an axial direction of the base (100), the groove (101) having an opening toward a first direction and configured for allowing an implant to be accommodated and placed therein;
the first workpiece (200) comprising a first inner cavity extending through the first workpiece (200), the first inner cavity having a first end hole (201) that has an inner diameter smaller than a maximum inner diameter of the groove (101); the first workpiece (200) configured to detachably connect to the base (100), and the first end hole (201) facing toward the first direction;
the second workpiece (300) comprising a second inner cavity extending through the second workpiece (300), the second inner cavity having a second end hole (301) that has an inner diameter smaller than the inner diameter of the first end hole (201); the second workpiece (300) configured to detachably connect to the base (100) or the first workpiece (200), and the second end hole (301) facing toward the first direction;
wherein the groove (101), the first end hole (201) and the second end hole (301) are coaxially arranged in sequence in the first direction after being assembled and connected together.

2. The loading tool for an implant according to claim 1, wherein the groove (101) comprises an outer sidewall (102) extending along the first direction; the base (100) comprising a sheath passageway (103) extending through the base (100) in the axial direction, the sheath passageway (103) arranged internal to and coaxially with the outer sidewall (102).

3. The loading tool for an implant according to claim 1, wherein the first inner cavity comprises a first neck section (202) tapered from an end thereof for connecting with the base (100) to the first end hole (201).

4. The loading tool for an implant according to claim 1, wherein the second inner cavity comprises a second neck section (302) tapered from an end thereof for connecting with the base (100) or the first workpiece (200) to the second end hole (301).

5. The loading tool for an implant according to claim 1, wherein one of the first workpiece (200) and the base (100) comprises a first fastener (108, 203), and the other one of the first workpiece (200) and the base (100) comprises a first receptacle (106, 205) matched with the first fastener (108, 203), and wherein the first workpiece (200) is connected to the base (100) by a snap-in connection between the first fastener (108, 203) and the first receptacle (106, 205).

6. The loading tool for an implant according to claim 5, wherein the first workpiece (200) comprises two or more first fasteners (108) distributed in a circumferential direction thereof, each of the first fasteners (108) extending to an end for connecting with the base (100); wherein the base (100) comprises two or more first receptacles (106) distributed in a circumferential direction of the base (100), each of the first receptacles (106) extending through the base (100) along the axial direction, so as to allow a corresponding one of the first fasteners (108) to pass therethrough for snap-in connection; a circumferential width of the first receptacle (106) is matched with the first fastener (108).

7. The loading tool for an implant according to claim 6, wherein the first workpiece (200) comprises two or more first guiding members (204) distributed in the circumferential direction of the first workpiece (200), each of the first guiding members (204) extending to an end for connecting with the base (100); the first receptacle (106) further configured to allow the first guiding member (204) to pass therethrough for snap-in connection; the circumferential width of the first receptacle (106) matched with the first guiding member (204).

8. The loading tool for an implant according to claim 5, wherein the first fastener (203) protrudes from the base (100) in a radial direction thereof, and the first receptacle (205) is formed on the first workpiece (200) and has an L-shape.

9. The loading tool for an implant according to claim 1, wherein the second workpiece (300) comprises a second fastener (206, 303), and the base (100) or the first workpiece (200) comprises a second receptacle (107, 305) matched with the second fastener (206, 303); or, the base (100) or the first workpiece (200) comprises a second fastener (206, 303), and the second workpiece (300) comprises a second receptacle (107, 305) matched with the second fastener (206, 303); the second workpiece (300) connected to the base (100) or the first workpiece (200) by a snap-in connection between the second fastener (206, 303) and the second receptacle (107, 305).

10. The loading tool for an implant according to claim 9, wherein the second workpiece (300) comprises two or more second fasteners (303) distributed in a circumferential direction of the second workpiece (300), each of the second fasteners (303) extending to an end for connecting with the base (100); wherein the base (100) comprises two or more second receptacles (107) distributed in a circumferential direction of the base (100), each of the second receptacles (107) extending through the base (100) along the axial direction thereof, so as to allow a corresponding one of the second fasteners (303) to pass therethrough for snap-in connection; a circumferential width of the second receptacle (107) matched with the second fastener (303).

11. The loading tool for an implant according to claim 10, wherein the second workpiece (300) comprises two or more second guiding members (304) distributed in the circumferential direction of the second workpiece (300), each of the second guiding members (304) extending to an end for connecting with the base (100); the second receptacle (107) further configured to allow the second guiding member (304) to pass therethrough for snap-in connection; the circumferential width of the second receptacle (107) matched with the second guiding member (304).

12. The loading tool for an implant according to claim 9, wherein the second fastener (206) protrudes from the first workpiece (200) in a radial direction thereof, and the second receptacle (305) is formed on the second workpiece (300) and has an L-shape.

13. The loading tool for an implant according to claim 1, wherein the first workpiece (200) is connected with the base (100) by threaded engagement; or, the second workpiece (300) is connected with the base (100) or the first workpiece (200) by threaded engagement.

14. The loading tool for an implant according to claim 1, further comprising a diameter per-reducing workpiece (400) that comprises a third inner cavity extending through the diameter per-reducing workpiece (400), the third inner cavity having a third end hole and a fourth end hole (402) opposite to the third end hole, the third end hole having an inner diameter that is smaller than an inner diameter of the fourth end hole (402) and not larger than the maximum inner diameter of the groove (101); the diameter per-reducing workpiece (400) configured to pre-reduce a diameter of the implant; the third inner cavity comprising an inlet section, a buffer section (403) and an outlet section, which are arranged in sequence from the fourth end hole (402) to the third end hole (401); the buffer section (403) having a drum shape.

15. A loading system for an implant, comprising:
a delivery device configured to deliver an implant to a target position; and
the loading tool for an implant according to any one of claims 1 to 14, configured to load the implant into the delivery device.

## Patentansprüche

1. Ladewerkzeug für ein Implantat, das eine Basis (100), ein erstes Werkstück (200) und ein zweites Werkstück (300) umfasst;
wobei die Basis (100) eine Nut (101) umfasst, die entlang einer axialen Richtung der Basis (100) angeordnet ist, wobei die Nut (101) eine Öffnung in eine erste Richtung aufweist und so konfiguriert ist, dass ein Implantat darin untergebracht und platziert werden kann;
das erste Werkstück (200) einen ersten inneren Hohlraum umfasst, der sich durch das erste Werkstück (200) erstreckt, wobei der erste innere Hohlraum ein erstes Endloch (201) aufweist, das einen Innendurchmesser hat, der kleiner ist als ein maximaler Innendurchmesser der Nut (101); das erste Werkstück (200) so konfiguriert ist, dass es lösbar mit der Basis (100) verbunden ist, und das erste Endloch (201) in die erste Richtung weist;
das zweite Werkstück (300) einen zweiten inneren Hohlraum umfasst, der sich durch das zweite Werkstück (300) erstreckt, wobei der zweite innere Hohlraum ein zweites Endloch (301) aufweist, das einen Innendurchmesser hat, der kleiner ist als der Innendurchmesser des ersten Endlochs (201); das zweite Werkstück (300) so konfiguriert ist, dass es lösbar mit der Basis (100) oder dem ersten Werkstück (200) verbunden ist, und das zweite Endloch (301) in die erste Richtung weist;
wobei die Nut (101), das erste Endloch (201) und das zweite Endloch (301) nach dem Zusammenbau und der Verbindung miteinander in der ersten Richtung koaxial hintereinander angeordnet sind.

2. Ladewerkzeug für ein Implantat nach Anspruch 1, wobei die Nut (101) eine äußere Seitenwand (102) aufweist, die sich entlang der ersten Richtung erstreckt; wobei die Basis (100) einen Hülsendurchgang (103) aufweist, der sich durch die Basis (100) in der axialen Richtung erstreckt, wobei der Hülsendurchgang (103) innerhalb und koaxial mit der äußeren Seitenwand (102) angeordnet ist.

3. Ladewerkzeug für ein Implantat nach Anspruch 1, wobei der erste innere Hohlraum einen ersten Halsabschnitt (202) umfasst, der sich von einem Ende desselben zur Verbindung mit der Basis (100) zum ersten Endloch (201) verjüngt.

4. Ladewerkzeug für ein Implantat nach Anspruch 1, wobei der zweite innere Hohlraum einen zweiten Halsabschnitt (302) umfasst, der sich von einem Ende desselben verjüngt, um mit der Basis (100) oder dem ersten Werkstück (200) zum zweiten Endloch (301) verbunden zu werden.

5. Ladewerkzeug für ein Implantat nach Anspruch 1, wobei eines von erstem Werkstück (200) oder Basis (100) ein erstes Befestigungselement (108, 203) umfasst, und das andere von erstem Werkstück (200) und Basis (100) eine erste Aufnahme (106, 205) umfasst, die mit dem ersten Befestigungselement (108, 203) zusammenpasst, und wobei das erste Werkstück (200) mit der Basis (100) durch eine Einrastverbindung zwischen dem ersten Befestigungselement (108, 203) und der ersten Aufnahme (106, 205) verbunden ist.

6. Ladewerkzeug für ein Implantat nach Anspruch 5, wobei das erste Werkstück (200) zwei oder mehr erste Befestigungselemente (108) aufweist, die in einer Umfangsrichtung desselben verteilt sind, wobei sich jedes der ersten Befestigungselemente (108) zu einem Ende zum Verbinden mit der Basis (100) erstreckt; wobei die Basis (100) zwei oder mehr erste Aufnahmen (106) umfasst, die in einer Umfangsrichtung der Basis (100) verteilt sind, wobei sich jede der ersten Aufnahmen (106) durch die Basis (100) entlang der axialen Richtung erstreckt, um zu ermöglichen, dass ein entsprechendes der ersten Befestigungselemente (108) für eine Einrastverbindung durch sie hindurchgeht; wobei eine Umfangsbreite der ersten Aufnahme (106) an das erste Befestigungselement (108) angepasst ist.

7. Ladewerkzeug für ein Implantat nach Anspruch 6, wobei das erste Werkstück (200) zwei oder mehr erste Führungselemente (204) umfasst, die in der Umfangsrichtung des ersten Werkstücks (200) verteilt sind, wobei sich jedes der ersten Führungselemente (204) zu einem Ende zum Verbinden mit der Basis (100) erstreckt; wobei die erste Aufnahme (106) ferner so konfiguriert ist, dass sie es dem ersten Führungselement (204) ermöglicht, zum Einrasten durch sie hindurchzugehen; wobei die Umfangsbreite der ersten Aufnahme (106) an das erste Führungselement (204) angepasst ist.

8. Ladewerkzeug für ein Implantat nach Anspruch 5, wobei das erste Befestigungselement (203) von der Basis (100) in einer radialen Richtung derselben vorsteht und die erste Aufnahme (205) an dem ersten Werkstück (200) ausgebildet ist und eine L-Form aufweist.

9. Ladewerkzeug für ein Implantat nach Anspruch 1, wobei das zweite Werkstück (300) ein zweites Befestigungselement (206, 303) umfasst, und die Basis (100) oder das erste Werkstück (200) eine zweite Aufnahme (107, 305) umfasst, die mit dem zweiten Befestigungselement (206, 303) zusammenpasst; oder die Basis (100) oder das erste Werkstück (200) ein zweites Befestigungselement (206, 303) umfasst, und das zweite Werkstück (300) eine zweite Aufnahme (107, 305) umfasst, die mit dem zweiten Befestigungselement (206, 303) zusammenpasst; wobei das zweite Werkstück (300) mit der Basis (100) oder dem ersten Werkstück (200) durch eine Einrastverbindung zwischen dem zweiten Befestigungselement (206, 303) und der zweiten Aufnahme (107, 305) verbunden ist.

10. Ladewerkzeug für ein Implantat nach Anspruch 9, wobei das zweite Werkstück (300) zwei oder mehr zweite Befestigungselemente (303) umfasst, die in einer Umfangsrichtung des zweiten Werkstücks (300) verteilt sind, wobei sich jedes der zweiten Befestigungselemente (303) zu einem Ende zum Verbinden mit der Basis (100) erstreckt; wobei die Basis (100) zwei oder mehr zweite Aufnahmen (107) umfasst, die in einer Umfangsrichtung der Basis (100) verteilt sind, wobei sich jede der zweiten Aufnahmen (107) durch die Basis (100) entlang der axialen Richtung derselben erstreckt, um zu ermöglichen, dass ein entsprechendes der zweiten Befestigungsmittel (303) für eine Einrastverbindung durch sie hindurchgeht; wobei eine Umfangsbreite der zweiten Aufnahme (107) an das zweite Befestigungsmittel (303) angepasst ist.

11. Ladewerkzeug für ein Implantat nach Anspruch 10, wobei das zweite Werkstück (300) zwei oder mehr zweite Führungselemente (304) umfasst, die in der Umfangsrichtung des zweiten Werkstücks (300) verteilt sind, wobei sich jedes der zweiten Führungselemente (304) zu einem Ende zum Verbinden mit der Basis (100) erstreckt; wobei die zweite Aufnahme (107) ferner so konfiguriert ist, dass sie es dem zweiten Führungselement (304) ermöglicht, für eine Einrastverbindung durch sie hindurchzugehen; wobei die Umfangsbreite der zweiten Aufnahme (107) an das zweite Führungselement (304) angepasst ist.

12. Ladewerkzeug für ein Implantat nach Anspruch 9, wobei das zweite Befestigungselement (206) aus dem ersten Werkstück (200) in dessen radialer Richtung hervorragt und die zweite Aufnahme (305) an dem zweiten Werkstück (300) ausgebildet ist und eine L-Form aufweist.

13. Ladewerkzeug für ein Implantat nach Anspruch 1, wobei das erste Werkstück (200) mit der Basis (100) durch Gewindeeingriff verbunden ist; oder das zweite Werkstück (300) mit der Basis (100) oder dem ersten Werkstück (200) durch Gewindeeingriff verbunden ist.

14. Ladewerkzeug für ein Implantat nach Anspruch 1, ferner umfassend ein durchmesserreduzierendes Werkstück (400), das einen dritten inneren Hohlraum umfasst, der sich durch das durchmesserreduzierende Werkstück (400) erstreckt, wobei der dritte innere Hohlraum ein drittes Endloch und ein viertes Endloch (402) gegenüber dem dritten Endloch aufweist, wobei das dritte Endloch einen Innendurchmesser aufweist, der kleiner als ein Innendurchmesser des vierten Endlochs (402) und nicht größer als der maximale Innendurchmesser der Nut (101) ist; das durchmesserverringernde Werkstück (400) so konfiguriert ist, dass es einen Durchmesser des Implantats vorverringert; der dritte innere Hohlraum einen Einlassabschnitt, einen Pufferabschnitt (403) und einen Auslassabschnitt umfasst, die nacheinander von dem vierten Endloch (402) zu dem dritten Endloch (401) angeordnet sind; wobei der Pufferabschnitt (403) eine Trommelform aufweist.

15. Ein Ladesystem für ein Implantat, das Folgendes umfasst:
eine Einführvorrichtung, die so konfiguriert ist, dass sie ein Implantat an eine Zielposition bringt; und
das Ladewerkzeug für ein Implantat nach einem der Ansprüche 1 bis 14, das so konfiguriert ist, dass es das Implantat in die Einführvorrichtung lädt.

## Revendications

1. Outil de chargement pour un implant, comprenant une base (100), une première pièce (200) et une deuxième pièce (300) ;
dans lequel la base (100) comprend une rainure (101) disposée le long d'une direction axiale de la base (100), la rainure (101) présentant une ouverture vers une première direction et configurée pour permettre à un implant d'être logé et placé à l'intérieur ;
la première pièce (200) comprenant une première cavité intérieure s'étendant à travers la première pièce (200), la première cavité intérieure étant dotée d'un premier trou d'extrémité (201) qui présente un diamètre intérieur inférieur à un diamètre intérieur maximal de la rainure (101) ; la première pièce (200) étant configurée pour se connecter de manière amovible à la base (100), et le premier trou d'extrémité (201) étant orienté vers la première direction ;
la deuxième pièce (300) comprenant une deuxième cavité intérieure s'étendant à travers la deuxième pièce (300), la deuxième cavité intérieure étant dotée d'un deuxième trou d'extrémité (301) qui présente un diamètre intérieur inférieur au diamètre du premier trou d'extrémité (201) ; la deuxième pièce (300) étant configurée pour se connecter de manière amovible à la base (100), ou la première pièce (200) et le deuxième trou d'extrémité (301) étant orienté(e) vers la première direction ;
dans lequel la rainure (101), le premier trou d'extrémité (201) et le deuxième trou d'extrémité (301) sont disposés coaxialement en séquence dans la première direction après avoir été assemblés et connectés ensemble.

2. Outil de chargement pour un implant selon la revendication 1, dans lequel la rainure (101) comprend une paroi latérale extérieure (102) s'étendant le long de la première direction ; la base (100) comprenant un passage de gaine (103) s'étendant à travers la base (100) dans la direction axiale, le passage de gaine (103) étant disposé à l'intérieur de la paroi latérale extérieure (102) et coaxialement à celle-ci.

3. Outil de chargement pour un implant selon la revendication 1, dans lequel la première cavité interne comprend une première section de col (202) effilée à partir d'une de ses extrémités pour se connecter à la base (100) jusqu'au premier trou d'extrémité (201).

4. Outil de chargement pour un implant selon la revendication 1, dans lequel la deuxième cavité interne comprend une deuxième section de col (302) effilée à partir d'une de ses extrémités pour se connecter à la base (100) ou à la première pièce (200) jusqu'au deuxième trou d'extrémité (301).

5. Outil de chargement d'un implant selon la revendication 1, dans lequel l'une de la première pièce (200) et de la base (100) comprend une première fixation (108, 203), et l'autre de la première pièce (200) et de la base (100) comprend un premier réceptacle (106, 205) adapté à la première fixation (108, 203), et dans lequel la première pièce (200) est reliée à la base (100) par une connexion par encliquetage entre la première fixation (108, 203) et le premier réceptacle (106, 205).

6. Outil de chargement d'un implant selon la revendication 5, dans lequel la première pièce (200) comprend deux ou plusieurs premières fixations (108) réparties dans une direction circonférentielle de celle-ci, chacune des premières fixations (108) s'étendant jusqu'à une extrémité pour se connecter à la base (100) ; dans lequel la base (100) comprend deux ou plusieurs premiers réceptacles (106) répartis dans la direction circonférentielle de la base (100), chacun des premiers réceptacles (106) s'étendant à travers la base (100) le long de la direction axiale, de manière à permettre à l'une des premières fixations (108) correspondante de passer à travers elle pour une connexion par encliquetage ; une largeur circonférentielle du premier réceptacle (106) est adaptée à la première fixation (108).

7. Outil de chargement pour un implant selon la revendication 6, dans lequel la première pièce (200) comprend deux ou plusieurs premiers éléments de guidage (204) répartis dans la direction circonférentielle de la première pièce (200), chacun des premiers éléments de guidage (204) s'étendant jusqu'à une extrémité pour se connecter à la base (100) ; le premier réceptacle (106) est en outre configuré pour permettre au premier élément de guidage (204) de le traverser pour une connexion par encliquetage ; la largeur circonférentielle du premier réceptacle (106) est adaptée au premier élément de guidage (204).

8. Outil de chargement d'un implant selon la revendication 5, dans lequel le premier élément de fixation (203) fait saillie de la base (100) dans une direction radiale, et le premier réceptacle (205) est formé sur la première pièce (200) et présente une forme en L.

9. Outil de chargement d'un implant selon la revendication 1, dans lequel la deuxième pièce (300) comprend une deuxième fixation (206, 303), et la base (100) ou la première pièce (200) comprend un deuxième réceptacle (107, 305) adapté à la deuxième fixation (206, 303) ; ou, la base (100) ou la première pièce (200) comprend une deuxième fixation (206, 303), et la deuxième pièce (300) comprend un deuxième réceptacle (107, 305) adapté à la deuxième fixation (206, 303) ; la deuxième pièce (300) est reliée à la base (100) ou à la première pièce (200) par une liaison par encliquetage entre la deuxième fixation (206, 303) et le deuxième réceptacle (107, 305).

10. Outil de chargement d'un implant selon la revendication 9, dans lequel la deuxième pièce (300) comprend deux ou plusieurs deuxièmes fixations (303) réparties dans une direction circonférentielle de la deuxième pièce (300), chacune des deuxièmes fixations (303) s'étendant jusqu'à une extrémité pour se connecter à la base (100) ; dans lequel la base (100) comprend deux ou plusieurs deuxièmes réceptacles (107) répartis dans une direction circonférentielle de la base (100), chacun des deuxièmes réceptacles (107) s'étendant à travers la base (100) le long de la direction axiale, de manière à permettre à l'une des deuxièmes fixations (303) correspondante de passer à travers elle pour une connexion par encliquetage ; une largeur circonférentielle du deuxième réceptacle (107) est adaptée à la deuxième fixation (303).

11. Outil de chargement pour un implant selon la revendication 10, dans lequel la deuxième pièce (300) comprend deux ou plusieurs deuxièmes éléments de guidage (304) répartis dans la direction circonférentielle de la deuxième pièce (300), chacun des deuxièmes éléments de guidage (304) s'étendant jusqu'à une extrémité pour se connecter à la base (100) ; le deuxième réceptacle (107) est en outre configuré pour permettre au deuxième élément de guidage (304) de le traverser pour une connexion par encliquetage ; la largeur circonférentielle du deuxième réceptacle (107) est adaptée au deuxième élément de guidage (304).

12. Outil de chargement d'un implant selon la revendication 9, dans lequel le deuxième élément de fixation (206) fait saillie de la première pièce (200) dans une direction radiale, et le deuxième réceptacle (305) est formé sur la deuxième pièce (300) et présente une forme en L.

13. Outil de chargement d'un implant selon la revendication 1, dans lequel la première pièce (200) est reliée à la base (100) par un engagement fileté ; ou, la deuxième pièce (300) est reliée à la base (100) ou à la première pièce (200) par un engagement fileté.

14. Outil de chargement pour un implant selon la revendication 1, comprenant en outre une pièce de réduction du diamètre (400) qui comprend une troisième cavité intérieure s'étendant à travers la pièce de réduction du diamètre (400), la troisième cavité intérieure présentant un troisième trou d'extrémité et un quatrième trou d'extrémité (402) opposé au troisième trou d'extrémité, le troisième trou d'extrémité présentant un diamètre intérieur qui est inférieur à un diamètre intérieur du quatrième trou d'extrémité (402) et pas plus grand que le diamètre intérieur maximum de la rainure (101) ; la pièce de réduction du diamètre (400) étant configurée pour réduire le diamètre de l'implant ; la troisième cavité interne comprenant une section d'entrée, une section tampon (403) et une section de sortie, qui sont disposées en séquence du quatrième trou d'extrémité (402) au troisième trou d'extrémité (401) ; la section tampon (403) présentant la forme d'un tambour.

15. Système de chargement pour un implant, comprenant :
un dispositif de distribution configuré pour distribuer un implant dans une position cible ; et
l'outil de chargement d'un implant selon l'une quelconque des revendications 1 à 14, configuré pour charger l'implant dans le dispositif d'administration.
